# EUROPEAN PATENT APPLICATION

(11) **EP 4 082 997 A1**
(43) Date of publication of application: **02.11.2022**
(21) Application number: 20906400.5
(22) Date of filing: 13.08.2020
(51) Int. Cl.: C07C 21/18, C07C 17/25

(54) **ALKENE MANUFACTURING METHOD**

(30) Priority: 27.12.2019 JP 2019238725
(71) Applicant: Kureha Corporation, Chuo-ku Tokyo 103-8552 (JP)
(72) Inventor: SUZUKI, Kensuke, Tokyo 103-8552 (JP); YOSHIDA, Katsumi, Tokyo 103-8552 (JP); TOHMIYA, Hiraku, Tokyo 103-8552 (JP)
(74) Representative: Zimmermann & Partner Patentanwälte mbB
(86) International application number: PCT/JP2020/030816
(87) International publication number: WO 2021/131143

(57) **Abstract**

An object of the present invention is to provide a method of producing an alkene using a solvent that has little decrease in production rate over time and is easy to recover and recycle.

The method of producing an alkene, which solves the problem, includes: a step of contacting a solution containing an alcohol having three or more carbon atoms and a hydroxide of an alkali metal and/or an alkaline earth metal with a halogenated alkane.

## Description

### TECHNICAL FIELD

The present invention relates to a method of producing an alkene.

### BACKGROUND ART

As a method of producing an alkene, a production method of eliminating a hydrogen halide from a halogenated alkane that is substituted with a plurality of halogen atoms has been known. For example, Patent Document 1 discloses a method of obtaining vinylidene fluoride by dehydrochlorination of 1,1-difluoro-1-chloroethane under pressurized conditions in the presence of an inorganic alkali aqueous solution such as a potassium hydroxide aqueous solution and a phase transfer catalyst (tetrabutylammonium bromide, TBAB).

Also disclosed in Patent Document 2 is a method of obtaining vinylidene fluoride by alkaline degradation of 1,1-difluoro-1-chloroethane in a solution with dimethyl sulfoxide (DMSO) as a main solvent, and water, ethanol, diglyme, or N,N-dimethylformamide (DMF) as an auxiliary solvent.

### CITATION LIST

### Patent Document

Patent Document 1: CN 105384596 A
Patent Document 2: DE 1959343 A

### SUMMARY OF INVENTION

### Technical Problem

However, in the method of Patent Document 1, the phase transfer catalyst disappears by decomposition under basic conditions (Hofmann elimination). Therefore, a decrease in dehydrochlorination reaction rate occurs over time. In addition, an alkali metal salt, which is a byproduct, dissolves in the aqueous solution. When the amount of the alkali metal salt is increased, the inorganic alkali involved in the dehydrochlorination reaction is less likely to dissolve in the aqueous alkali solution. That is, the desired reaction is difficult to proceed.

On the other hand, in the method of Patent Document 2, no phase transfer catalyst is used, and thus the rate does not decrease due to disappearance of the phase transfer catalyst. However, from the perspective of production costs, the recovery and recycle of the solvent are desired, but DMSO degrades at a temperature near boiling point. Therefore, it is difficult to recover the solvent by distillation operation. Also, when water is used as the auxiliary solvent of the method, a byproduct salt dissolves therein. Also in this case, when the dissolved amount of the byproduct salt increases, a decrease in dehydrochlorination reaction rate tends to occur.

The present invention has been made in view of the above problems. Specifically, an object of the present invention is to provide a method of producing an alkene using a solvent that has little decrease in formation rate over time and is easy to recover and recycle.

### SOLUTION TO PROBLEM

To solve the above problems, the following method of producing an alkene is provided.

A method of producing an alkene, including:
a step of contacting a solution containing an alcohol having three or more carbon atoms and a hydroxide of an alkali metal and/or an alkaline earth metal with a halogenated alkane.

### Advantageous Effects of Invention

According to the production method of the present invention, no phase transfer catalyst need to be used, and the byproduct salt does not easily dissolve in the reaction solution. This makes it difficult to reduce the reaction rate and possible to efficiently produce an alkene. Furthermore, the solvents used in the production of an alkene can also be recovered.

### DESCRIPTION OF EMBODIMENTS

The method of producing an alkene of the present invention includes: a step of contacting a solution containing an alcohol having three or more carbon atoms and a hydroxide of an alkali metal and/or an alkaline earth metal (hereinafter also referred to as "alkali-based compound") with a halogenated alkane.

The alkali-based compound is generally difficult to dissolve in organic solvents. On the other hand, the halogenated alkane is difficult to dissolve in aqueous solutions and the like. Therefore, a phase transfer catalyst has hitherto been important for sufficiently contacting and reacting the alkali-based compound with the halogenated alkane. However, when the phase transfer catalyst is used, the reaction rate is likely to decrease due to disappearance of the phase transfer catalyst. Also, DMSO, which is known as a solvent capable of dissolving the alkali-based compound and the halogenated alkane, is difficult to recover after use. Furthermore, when water is used with DMSO, there is a problem that the byproduct salt generated by the reaction dissolves in the solution, which leads to a decrease in reaction rate.

In contrast, the present inventors have found that both the alkali-based compound and the halogenated alkane can be dissolved in an alcohol having three or more carbon atoms, and, further, that they are efficiently reacted in a solution containing the alcohol having three or more carbon atoms. Additionally, when the solution used in the reaction contains an alcohol having three or more carbon atoms, it is not necessary to use water as an auxiliary solvent. In other words, a salt (alkali metal salt or alkaline earth metal salt) that is by-produced by a reaction of an alkali-based compound and a halogenated alkane does not easily dissolve in the solution. Therefore, even when the amount of the byproduct salt is large, solubility of the alkali-based compound relative to the reaction solution is unlikely to change, which enables efficient alkene production. Hereinafter, one embodiment of the method will be described as an example in detail below.

In the method of producing an alkene according to the present embodiment, an alcohol having three or more carbon atoms and an alkali-based compound are incorporated in a solution (liquid phase) used in a reaction. On the other hand, a halogenated alkane is incorporated in a gas phase. Then, for example, by contacting the gas phase containing the halogenated alkane with the solution (liquid phase), the halogenated alkane is dissolved in the solution, the alkali-based compound and the halogenated alkane are reacted, and an alkene is formed.

Here, the solution used in production of an alkene may contain an alcohol having three or more carbon atoms and an alkali-based compound, and may contain a solvent other than the alcohol having three or more carbon atoms. However, the amount of water in the solution is preferably small enough. The amount of water is preferably 1 part by mass or less, more preferably 0.5 parts by mass or less, and particularly preferably 0.1 parts by mass or less, with respect to a total amount, 100 parts by mass, of the solution. When the amount of water is small, the byproduct salt is less likely to dissolve in the solution, and solubility of the alkali-based compound is less likely to change.

In addition, when the solution contains any other solvent, the alcohol having three or more carbon atoms may be a main solvent or an auxiliary solvent. Note that, in the present specification, the main solvent refers to a solvent having the highest content among a plurality of solvents, and the auxiliary solvent refers to a solvent other than the main solvent.

The amount of the alcohol having three or more carbon atoms in the solution is preferably from 1 to 100 parts by mass, more preferably from 20 to 100 parts by mass, and even more preferably from 50 to 100 parts by mass, with respect to a total amount, 100 parts by mass, of the solvent. When the amount of alcohol having three or more carbon atoms is within this range, it is possible to sufficiently dissolve the alkali-based compound and the halogenated alkane in the solution, and to efficiently produce an alkene.

Here, the alcohol having three or more carbon atoms, in the present specification, refers to a compound having a saturated or unsaturated aliphatic hydrocarbon chain having three or more carbon atoms or a saturated or unsaturated alicyclic hydrocarbon chain having three or more carbon atoms, and one or more hydroxy groups bonded thereto. Also, a compound having a linking group such as an ether bond among a plurality of aliphatic hydrocarbon and/or alicyclic hydrocarbon chains (e.g., glycol ether) is also handled as an alcohol. On the other hand, a compound in which a hydroxy group is bonded to an aromatic ring such as phenol (generally, a compound referred to as phenol) is not included in the alcohol having three or more carbon atoms. Furthermore, the alcohol having three or more carbon atoms may be a primary, secondary, or tertiary alcohol, but is more preferably a secondary or tertiary alcohol. When the alcohol having three or more carbon atoms is a secondary or tertiary alcohol, formation of byproducts is likely to be suppressed.

Here, the alcohol having three or more carbon atoms is preferably water soluble. The alcohol having three or more carbon atoms, when having water solubility, easily dissolves the alkali-based compound. Note that, in the present specification, water solubility refers to solubility in water of 2 g/100 mL or more, at normal temperature (25°C) and ambient pressure.

Furthermore, the alcohol having three or more carbon atoms may be a monohydric alcohol or may be a dihydric or higher alcohol. When the alcohol having three or more carbon atoms is a monohydric alcohol, the number of carbon atoms is preferably 3 or more and 7 or less, and more preferably 3 or more and 5 or less. When the number of carbon atoms is within the range, the alcohol easily attains water solubility, and easily dissolves the alkali-based compound and the halogenated alkane.

Examples of the monohydric alcohol include saturated aliphatic hydrocarbon alcohols such as n-propanol, isopropanol, n-butanol, t-butyl alcohol, and n-pentyl alcohol; saturated alicyclic hydrocarbon alcohols such as cyclopentanol and cyclohexanol; and unsaturated aliphatic hydrocarbon alcohols such as allyl alcohol and 2-propyn-1-ol. All of them are water soluble. Among them, saturated aliphatic hydrocarbons are preferable, and aliphatic hydrocarbon alcohols having branched chains are particularly preferable. When the alcohol having three or more carbon atoms has a branched chain, the molecular structure thereof becomes bulky, and the formation of byproducts is likely to be suppressed.

Examples of the dihydric or higher alcohols include glycols such as trimethylene glycol, propylene glycol, diethylene glycol, triethylene glycol, tetraethylene glycol, pentaethylene glycol, hexaethylene glycol, diisopropylene glycol, dipropylene glycol, tripropylene glycol, tetrapropylene glycol, tetramethylene glycol, and polyethylene glycol; and triols such as glycerin and 1,2,4-butantriol. All of them are water soluble. Note that, glycols, when having a molecular weight of 2000 or less, are preferred from the perspective that the viscosity of the solution is not excessively increased, and further that the alkali-based compound is easily dissolved therein.

Furthermore, a solvent other than the above alcohols, which can be used in the solution in the present embodiment, is not particularly limited as long as it is a solvent that has miscibility with the alcohol having three or more carbon atoms and does not inhibit dissolution of the alkali-based compound. Note that the solvent is a solvent which can be uniformly mixed with the alcohol having three or more carbon atoms. Examples of the solvent include toluene, benzene, isopropyl benzene, o-xylene, m-xylene, p-xylene, o-dichlorobenzene, m-dichlorobenzene, p-dichlorobenzene, hexane, diethyl ether, and 1,4-dioxane.

The amount of the solvent other than the above alcohols is preferably 99 parts by mass or less, more preferably 80 parts by mass or less, and even more preferably 50 parts by mass or less, with respect to the total amount, 100 parts by mass, of the solvent. When the amount of the solvent other than the above alcohols is within this range, an alkene can be efficiently produced.

Examples of the alkali-based compound contained in the solution has only to be a hydroxide of an alkali metal and/or an alkaline earth metal, and include potassium hydroxide (KOH), sodium hydroxide (NaOH), lithium hydroxide (LiOH), magnesium hydroxide (Mg(OH)₂), and calcium hydroxide (Ca(OH)₂). Among them, potassium hydroxide (KOH) and sodium hydroxide (NaOH) are preferred, and sodium hydroxide (NaOH) is more preferred, from the perspective of the solubility in the alcohol having three or more carbon atoms, the reactivity with the halogenated alkane, and the like.

A higher content of the alkali-based compound in the liquid phase (solution) tends to more increase the alkene formation efficiency. The content of the alkali-based compound is appropriately selected depending on the type of alkali-based compound or the like. The content of the alkali-based compound in the solution is preferably from 0.5 to 5 mol, more preferably from 0.5 to 2.5 mol, and even more preferably from 1 to 2 mol, relative to 1 mol of the halogenated alkane (e.g., compound represented by General Formula (1) described later), from the perspective of increasing the efficiency of alkene formation while making difficult deterioration in reaction vessel, piping, and the like.

On the other hand, the gas phase at the time of alkene formation contains the halogenated alkane as a raw material, and, after the reaction proceeds, further contains an alkene as a reaction product.

The halogenated alkane is a molecule having at least one halogen atom and at least one hydrogen atom in one molecule and is a gas at normal temperature. The halogenated alkane forms an alkene by contact with the alkali-based compound in the liquid phase and elimination of the halogen atom with the hydrogen bonded to a carbon atom adjacent to the halogen atom as a hydrogen halide.

Note that the halogenated alkane may be a molecule having at least two halogen atoms and at least one hydrogen atom in one molecule and may be a gas at normal temperature. Such a halogenated alkane forms a halogenated alkene by contact with the alkali-based compound in the liquid phase and elimination of one of at least two halogen atoms (one having a smaller bond dissociation energy with a carbon atom) with the hydrogen bonded to the adjacent carbon atom as a hydrogen halide.

Examples of the halogen atom contained in the halogenated alkane include a fluorine (F) atom, a chlorine (CI) atom, a bromine (Br) atom, and an iodine (I) atom.

Examples of the halogenated alkane include fluoroethane, 1,1-difluoroethane, 1,1,1-trifluoroethane, 1,1,1,2-tetrafluoroethane, 1,1,2,2-tetrafluoroethane, 1,1,1,2,2-pentafluoroethane, chloroethane, 1,1-dichloroethane, 1,2-dichloroethane, 1,1,2-trichloroethane, 1,1-difluoro-1-chloroethane, 1,2-dichloropropane, 1,3-dichloropropane, 1,2,3-trichloropropane, 1,1,1,2,2-pentafluoropropane, 1,1,1,3,3-pentafluoropropane, 1,1,1,2,3,3-hexafluoropropane, 1,1,1,3,3,3-hexafluoropropane, 1,1,1,2,3,3,3-heptafluoropropane, 1,1,1,2-tetrafluoro-2-chloropropane, 1,1,1,2-tetrafluoro-3-chloropropane, 1,1,1,2,2-pentafluoro-3,3-dichloropropane, 1,1,1-trifluoro-2,2-dichloropentane, 1,1,1,2-tetrafluoro-2-chloropentane, 1,1,1,2,3-pentafluoropentane, 1,1,1,2-tetrafluoro-3-chloropentane, 1,1,1,3-tetrafluoro-3-chloropentane, 1,2-dichlorobutane, and 1,4-dichlorobutane.

Among these, 1,1-difluoro-1-chloroethane, 1,1,1,2-tetrafluoro-2-chloropropane, 1,1,1,2-tetrafluoro-3-chloropropane, 1,1,1,2,2-pentafluoropropane, and 1,1,1,2,2-pentafluoro-3,3-dichloropropane are preferred.

The halogenated alkane is preferably a halogenated alkane represented by General Formula (1).

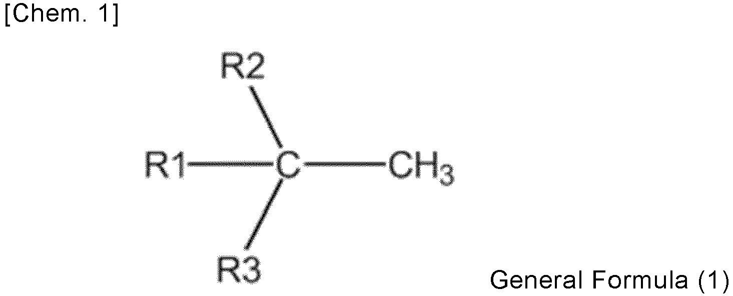

In General Formula (1), R1 represents a halogen atom, R2 represents a hydrogen atom, a halogen atom that is the same type as R1, or a halogen atom having a bond dissociation energy with a carbon atom greater than that of the atom represented by R1, R3 represents a halogen atom that is the same type as R1, a halogen atom having a bond dissociation energy with a carbon atom greater than that of the atom represented by R1, or an alkyl group having from 1 to 3 carbon atoms which may be substituted with any halogen atom.

From the halogenated alkane represented by General Formula (1), a halogenated alkene represented by General Formula (2) is formed by elimination of the hydrogen halide (R1-H).

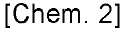

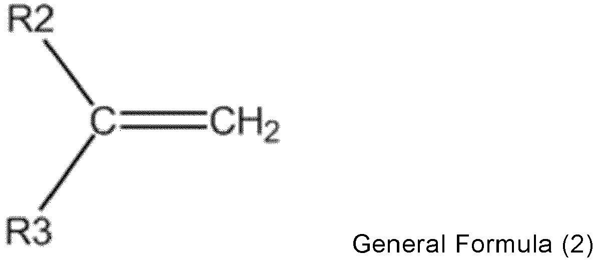

In General Formula (2), R2 is the same as R2 in General Formula (1) and represents a hydrogen atom or a halogen atom, R3 is the same as R3 in General Formula (1) and represents a halogen atom or an alkyl group having from 1 to 3 carbons which may be substituted with any halogen atom.

Note that, in General Formula (1) and General Formula (2), the halogen atom represented by R1, the halogen atoms represented by R2 and R3, and the halogen atom substituting for the alkyl group represented by R3 may be the same type or different type of atoms.

Furthermore, in General Formula (2), when R3 is an alkyl group substituted with a halogen atom, the alkyl group may be substituted with a plurality of halogen atoms, or all the hydrogens may be substituted with halogen atoms. At this time, the plurality of halogen atoms used for the substitution may be all the same type of atoms or may be a combination of different types of plurality of halogen atoms.

In General Formulas (1) and (2), R1 is preferably a fluorine (F) atom, a chlorine (CI) atom, or a bromine (Br) atom, and more preferably a chlorine (CI) atom or a bromine (Br) atom, and even more preferably a chlorine (CI) atom.

Furthermore, from the perspective of facilitating elimination of the hydrogen halide, in General Formula s(1) and (2), preferably R2 or R3 is a fluorine (F) atom, and more preferably both R2 and R3 are fluorine (F) atoms.

For example, the halogenated alkane can be 1,1-difluoro-1-chloroethane, and the halogenated alkene, which is the reaction product at this time, can be 1,1-difluoroethylene (vinylidene fluoride).

The content of the halogenated alkane in the reaction system is preferably from 5 to 100 parts by mass, more preferably from 10 to 50 parts by mass, and even more preferably from 10 to 25 parts by mass, with respect to the total amount, 100 parts by mass, of the solvent described above.

Note that the gas phase may contain an inert gas, such as a nitrogen (N₂) gas and an argon (Ar) gas; however, from the perspective of further enhancing the reaction efficiency, the gas phase preferably substantially only contains the halogenated alkane and the reaction product. "Substantially" means 99 vol% or greater of the gas phase is the halogenated alkane and the reaction product.

The method of producing an alkene described above has only to include a step of contacting the liquid phase with the gas phase.

Thereafter, the method of producing an alkene described above may further include a step of recovering the alkene, which is the reaction product, by separating the alkene from the liquid phase and the gas phase after the contact. The separation and recovery can be performed by known methods.

The method of producing an alkene described above can be performed, for example, by preparing the liquid phase by charging the alcohol and the alkali-based compound, and, optionally, any other solvent in a reaction vessel having an adequate capacity and then introducing the halogenated alkane in a gas form into the reaction vessel. Note that the treatment may be performed batchwise, or may be performed in a continuous manner.

The liquid phase may be prepared in the reaction vessel by charging the alcohol and the alkali-based compound in the reaction vessel, or may be prepared before charging of the reaction vessel. The order of these charging and mixing is not particularly limited.

Furthermore, it is preferable to discharge the gas component inside of the vessel by reducing the pressure inside the reaction vessel before the introduction of the halogenated alkane into the reaction vessel. After the pressure reduction, before the introduction of the halogenated alkane, the inert gas may be introduced into the reaction vessel.

After the introduction of the halogenated alkane, inside of the reaction vessel may be heated to promote the elimination reaction of the halogenated alkane. The temperature inside the reaction vessel at this time (reaction temperature) can be 20°C or higher and lower than 200°C, and is preferably from 20°C or higher and 140°C or lower, more preferably from 40°C or higher and 100°C or lower, and even more preferably from 40°C or higher and 80°C or lower.

Furthermore, the pressure inside the reaction vessel after the introduction of the halogenated alkane may be atmospheric pressure or higher and 5.0 MPa·G or lower, but preferably atmospheric pressure or higher and 2.0 MPa·G or lower, more preferably atmospheric pressure or higher and 1.0 MPa·G or lower, even more preferably 0.1 MPa·G or higher and 0.7 MPa·G or lower, and particularly preferably 0.1 MPa·G or higher and 0.5 MPa·G or lower.

Furthermore, the reaction time after the introduction of the halogenated alkane has only to be approximately 0.1 hours or longer and 8 hours or shorter.

After the reaction described above, a step of recovering, from the liquid phase, the alcohol having three or more carbon atoms from the solution may be further performed. A recovery method is not particularly limited, but may be, for example, a distillation method.

### [Examples]

Hereinafter, specific examples of the present invention will be described together with comparative examples, but the present invention is not limited thereto.

### Example 1

Into a 1-L pressure-resistant reaction vessel with an agitator (hereinafter simply referred to as "reaction vessel"), 281.1 g of n-propanol and 44.6 g of sodium hydroxide were charged and mixed together. The reaction vessel was completely tightly closed, the pressure inside the reaction vessel was reduced by a vacuum pump, and 55.0 g of 1,1-difluoro-1-chloroethane (R-142b) was filled in the reaction vessel. Stirring was started, and the temperature was increased to 80°C. After it was confirmed that the internal temperature reached 80°C, the temperature was maintained for 3 hours. The pressure inside the reaction vessel while the temperature was maintained was from 0.25 MPa·G to 0.46 MPa·G. After 3 hours, the heating was terminated. The reaction solution was cooled to 40°C or lower, and then the gas phase sample was collected in a gas collection bag. When the collected gas phase sample was analyzed by gas chromatography (the column used is CP-PoraPLOT Q ("PoraPLOT" is a trade name of Agilent Technologies, Inc.) available from this company), the conversion rate of 1,1-difluoro-1-chloroethane (R-142b) was 61.1%, and the selection rate of 1,1-difluoroethylene (VDF) was 76.2%. Note that the conversion rate is an amount of the reacted 1,1-difluoro-1-chloroethane, and the selection rate refers to a proportion of the reacted 1,1-difluoro-1-chloroethane which has been changed to the desired 1,1-difluoroethylene (VDF).

### Example 2

Into the reaction vessel described above, 273.3 g of isopropanol and 45.0 g of sodium hydroxide were charged and mixed together. The reaction vessel was completely tightly closed, the pressure inside the reaction vessel was reduced by a vacuum pump, and 55.0 g of 1,1-difluoro-1-chloroethane (R-142b) was filled in the reaction vessel. Stirring was started, and the temperature was increased to 80°C. After it was confirmed that the internal temperature reached 80°C, the temperature was maintained for 3 hours. The pressure inside the reaction vessel while the temperature was maintained was from 0.34 MPa·G to 0.51 MPa·G. After 3 hours, the heating was terminated. The reaction solution was cooled to 40°C or lower, and then the gas phase sample was collected in a gas collection bag. When the gas phase sample collected was analyzed by gas chromatography in the same manner as in Example 1, the conversion rate of 1,1-difluoro-1-chloroethane (R-142b) was 82.3%, and the selection rate of 1,1-difluoroethylene (VDF) was 94.3%.

### Example 3

Into the reaction vessel described above, 273.3 g of isopropanol and 44.6 g of sodium hydroxide were charged and mixed together. The reaction vessel was completely tightly closed, the pressure inside the reaction vessel was reduced by a vacuum pump, and 55.0 g of 1,1-difluoro-1-chloroethane (R-142b) was filled in the reaction vessel. Stirring was started, and the temperature was increased to 80°C. After it was confirmed that the internal temperature reached 80°C, the temperature was maintained for 0.25 hours. The pressure inside the reaction vessel while the temperature was maintained was from 0.35 MPa·G to 0.43 MPa·G. After 0.25 hours, the heating was terminated. The reaction solution was cooled to 40°C or lower, and then the gas phase sample was collected in a gas collection bag. When the gas phase sample collected was analyzed by gas chromatography in the same manner as in Example 1, the conversion rate of 1,1-difluoro-1-chloroethane (R-142b) was 42.5%, and the selection rate of 1,1-difluoroethylene (VDF) was 99.3%.

### Example 4

Into the reaction vessel described above, 273.3 g of isopropanol and 44.6 g of sodium hydroxide were charged and mixed together. The reaction vessel was completely tightly closed, the pressure inside the reaction vessel was reduced by a vacuum pump, and 55.0 g of 1,1-difluoro-1-chloroethane (R-142b) was filled in the reaction vessel. Stirring was started, and the temperature was increased to 60°C. After it was confirmed that the internal temperature reached 60°C, the temperature was maintained for 3 hours. The pressure inside the reaction vessel while the temperature was maintained was from 0.21 MPa·G to 0.34 MPa·G. After 3 hours, the heating was terminated. The reaction solution was cooled to 40°C or lower, and then the gas phase sample was collected in a gas collection bag. When the gas phase sample collected was analyzed by gas chromatography in the same manner as in Example 1, the conversion rate of 1,1-difluoro-1-chloroethane (R-142b) was 52.6%, and the selection rate of 1,1-difluoroethylene (VDF) was 98.1%.

### Example 5

Into the reaction vessel described above, 283.5 g of n-butanol and 44.6 g of sodium hydroxide were charged and mixed together. The reaction vessel was completely tightly closed, the pressure inside the reaction vessel was reduced by a vacuum pump, and 55.0 g of 1,1-difluoro-1-chloroethane (R-142b) was filled in the reaction vessel. Stirring was started, and the temperature was increased to 80°C. After it was confirmed that the internal temperature reached 80°C, the temperature was maintained for 3 hours. The pressure inside the reaction vessel while the temperature was maintained was from 0.33 MPa·G to 0.44 MPa·G. After 3 hours, the heating was terminated. The reaction solution was cooled to 40°C or lower, and then the gas phase sample was collected in a gas collection bag. When the gas phase sample collected was analyzed by gas chromatography in the same manner as in Example 1, the conversion rate of 1,1-difluoro-1-chloroethane (R-142b) was 38.5%, and the selection rate of 1,1-difluoroethylene (VDF) was 90.5%.

### Example 6

Into the reaction vessel described above, 273.4 g of t-butyl alcohol and 44.6 g of sodium hydroxide were charged and mixed together. The reaction vessel was completely tightly closed, the pressure inside the reaction vessel was reduced by a vacuum pump, and 55.0 g of 1,1-difluoro-1-chloroethane (R-142b) was filled in the reaction vessel. Stirring was started, and the temperature was increased to 80°C. After it was confirmed that the internal temperature reached 80°C, the temperature was maintained for 3 hours. The pressure inside the reaction vessel while the temperature was maintained was from 0.28 MPa·G to 0.41 MPa·G. After 3 hours, the heating was terminated. The reaction solution was cooled to 40°C or lower, and then the gas phase sample was collected in a gas collection bag. When the gas phase sample collected was analyzed by gas chromatography in the same manner as in Example 1, the conversion rate of 1,1-difluoro-1-chloroethane (R-142b) was 50.4%, and the selection rate of 1,1-difluoroethylene (VDF) was 99.9%.

### Example 7

Into the reaction vessel described above, 393.4 g of triethylene glycol and 11.2 g of sodium hydroxide were charged and mixed together. The reaction vessel was completely tightly closed, the pressure inside the reaction vessel was reduced by a vacuum pump, and 55.0 g of 1,1-difluoro-1-chloroethane (R-142b) was filled in the reaction vessel. Stirring was started, and the temperature was increased to 80°C. After it was confirmed that the internal temperature reached 80°C, the temperature was maintained for 3 hours. The pressure inside the reaction vessel while the temperature was maintained was from 0.59 MPa·G to 0.73 MPa·G. After 3 hours, the heating was terminated. The reaction solution was cooled to 40°C or lower, and then the gas phase sample was collected in a gas collection bag. When the gas phase sample collected was analyzed by gas chromatography in the same manner as in Example 1, the conversion rate of 1,1-difluoro-1-chloroethane (R-142b) was 49.6%, and the selection rate of 1,1-difluoroethylene (VDF) was 99.7%.

### Example 8

Into the reaction vessel described above, 395.5 g of Polyethylene Glycol 200 and 11.2 g of sodium hydroxide were charged and mixed together. The reaction vessel was completely tightly closed, the pressure inside the reaction vessel was reduced by a vacuum pump, and 55.0 g of 1,1-difluoro-1-chloroethane (R-142b) was filled in the reaction vessel. Stirring was started, and the temperature was increased to 80°C. After it was confirmed that the internal temperature reached 80°C, the temperature was maintained for 3 hours. The pressure inside the reaction vessel while the temperature was maintained was from 0.67 MPa·G to 0.70 MPa·G. After 3 hours, the heating was terminated. The reaction solution was cooled to 40°C or lower, and then the gas phase sample was collected in a gas collection bag. When the gas phase sample collected was analyzed by gas chromatography in the same manner as in Example 1, the conversion rate of 1,1-difluoro-1-chloroethane (R-142b) was 54.8%, and the selection rate of 1,1-difluoroethylene (VDF) was 100%.

### Example 9

Into the reaction vessel described above, 266.5 g of t-butyl alcohol, 10.0 g of Polyethylene Glycol 1000, and 44.6 g of sodium hydroxide were charged and mixed together. The reaction vessel was completely tightly closed, the pressure inside the reaction vessel was reduced by a vacuum pump, and 55.0 g of 1,1-difluoro-1-chloroethane (R-142b) was filled in the reaction vessel. Stirring was started, and the temperature was increased to 80°C. After it was confirmed that the internal temperature reached 80°C, the temperature was maintained for 3 hours. The pressure inside the reaction vessel while the temperature was maintained was from 0.32 MPa·G to 0.50 MPa·G. After 3 hours, the heating was terminated. The gas phase sample was collected in a gas collection bag. When the gas phase sample collected was analyzed by gas chromatography in the same manner as in Example 1, the conversion rate of 1,1-difluoro-1-chloroethane (R-142b) was 61.0%, and the selection rate of 1,1-difluoroethylene (VDF) was 100%.

### Example 10

Into the reaction vessel described above, 295.8 g of toluene, 10.0 g of Polyethylene Glycol 1000, and 44.6 g of sodium hydroxide were charged and mixed together. The reaction vessel was completely tightly closed, the pressure inside the reaction vessel was reduced by a vacuum pump, and 55.0 g of 1,1-difluoro-1-chloroethane (R-142b) was filled in the reaction vessel. Stirring was started, and the temperature was increased to 80°C. After it was confirmed that the internal temperature reached 80°C, the temperature was maintained for 3 hours. The pressure inside the reaction vessel while the temperature was maintained was from 0.24 MPa·G to 0.38 MPa·G. After 3 hours, the heating was terminated. The gas phase sample was collected in a gas collection bag. When the gas phase sample collected was analyzed by gas chromatography in the same manner as in Example 1, the conversion rate of 1,1-difluoro-1-chloroethane (R-142b) was 51.1%, and the selection rate of 1,1-difluoroethylene (VDF) was 100%.

### Comparative Example 1

Into the reaction vessel described above, 277.1 g of methanol and 46.4 g of sodium hydroxide were charged and mixed together. The reaction vessel was completely tightly closed, the pressure inside the reaction vessel was reduced by a vacuum pump, and 55.0 g of 1,1-difluoro-1-chloroethane (R-142b) was filled in the reaction vessel. Stirring was started, and the temperature was increased to 80°C. After it was confirmed that the internal temperature reached 80°C, the temperature was maintained for 3 hours. The pressure inside the reaction vessel while the temperature was maintained was from 0.47 MPa·G to 0.58 MPa·G. After 3 hours, the heating was terminated. The reaction solution was cooled to 40°C or lower, and then the gas phase sample was collected in a gas collection bag. When the gas phase sample collected was analyzed by gas chromatography in the same manner as in Example 1, the conversion rate of 1,1-difluoro-1-chloroethane (R-142b) was 58.5%, and the selection rate of 1,1-difluoroethylene (VDF) was 25.0%.

### Comparative Example 2

Into the reaction vessel described above, 276.2 g of ethanol and 45.0 g of sodium hydroxide were charged and mixed together. The reaction vessel was completely tightly closed, the pressure inside the reaction vessel was reduced by a vacuum pump, and 55.0 g of 1,1-difluoro-1-chloroethane (R-142b) was filled in the reaction vessel. Stirring was started, and the temperature was increased to 80°C. After it was confirmed that the internal temperature reached 80°C, the temperature was maintained for 3 hours. The pressure inside the reaction vessel while the temperature was maintained was from 0.27 MPa·G to 0.51 MPa·G. After 3 hours, the heating was terminated. The reaction solution was cooled to 40°C or lower, and then the gas phase sample was collected in a gas collection bag. When the gas phase sample collected was analyzed by gas chromatography in the same manner as in Example 1, the conversion rate of 1,1-difluoro-1-chloroethane (R-142b) was 74.6%, and the selection rate of 1,1-difluoroethylene (VDF) was 56.9%.

**Table 1**

| | | Example 1 | Example 2 | Example 3 | Example 4 | Example 5 | Example 6 | Example 7 |
|---|---|---|---|---|---|---|---|---|
| Alcohol | Type | n-PrOH | IPA | IPA | IPA | n-BuOH | t-BuOH | TEG |
| | Number of carbon atoms | 3 | 3 | 3 | 3 | 4 | 4 | 6 |
| | Mass [g] | 281.1 | 273.3 | 273.3 | 273.3 | 283.5 | 273.4 | 393.4 |
| Other solvents | Type | | | | | | | |
| | Mass [g] | | | | | | | |
| Alkali-based compound | Mass [g] | 44.6 | 45.0 | 44.6 | 44.6 | 44.6 | 44.6 | 11.2 |
| Halogenated alkane | Mass [g] | 55.0 | 55.0 | 55.0 | 55.0 | 55.0 | 55.0 | 55.0 |
| Conversion rate [%] | | 61.1 | 82.3 | 42.5 | 52.6 | 38.5 | 50.4 | 49.6 |
| Selection rate [%] | | 76.2 | 94.3 | 99.3 | 98.1 | 90.5 | 99.9 | 99.7 |

| | | | | | | | | |
|---|---|---|---|---|---|---|---|---|
| n-PrOH: n-propanol, IPA: isopropanol, n-BuOH: n-butanol, t-BuOH: t-butyl alcohol, TEG: triethylene glycol | | | | | | | | |

**[Table 2]**

| | | Example 8 | Example 9 | Example 10 | Comparative Example 1 | Comparative Example 2 |
|---|---|---|---|---|---|---|
| Alcohol 1 | Type | PEG200 | t-BuOH | PEG1000 | MeOH | EtOH |
| | Number of carbon atoms | 6< | 4 | 6< | 1 | 2 |
| | Mass [g] | 395.5 | 266.5 | 10.0 | 277.1 | 276.2 |
| Alcohol 2 | Type | | PEG1000 | | | |
| | Number of carbon atoms | | 6< | | | |
| | Mass [g] | | 10.0 | | | |
| Other solvents | Type | | | Toluene | | |
| | Mass [g] | | | 295.8 | | |
| Alkali-based compound | Mass [g] | 11.2 | 44.6 | 44.6 | 46.4 | 45.0 |
| Halogenated alkane | Mass [g] | 55.0 | 55.0 | 55.0 | 55.0 | 55.0 |
| Conversion rate [%] | | 54.8 | 61.0 | 51.1 | 58.5 | 74.6 |
| Selection rate [%] | | 100 | 100 | 100 | 25.0 | 56.9 |

| | | | | | | |
|---|---|---|---|---|---|---|
| t-BuOH: t-butyl alcohol, MeOH: methanol, EtOH: ethanol, PEG200: Polyethylene Glycol 200, PEG1000: Polyethylene Glycol 1000 | | | | | | |

As shown in Tables 1 and 2 above, the selection rate of 1,1-difluoroethylene (VDF) was very high when the solution containing the alcohol having three or more carbon atoms and the alkali-based compound was brought into contact with the halogenated alkane (Examples 1 to 10). It can be said that the desired reaction occurred in the solution since the alcohol dissolved both the alkali-based compound and the halogenated alkane. In addition, it can be said that the reaction rate was less likely to decrease, since water was not used as a solvent, in these Examples. On the other hand, when the number of carbon atoms of the alcohol is less than 3, a sufficient selection rate could not be obtained (Comparative Examples 1 and 2).

When the main chain of the alcohol was branched, or when the alcohol is a secondary alcohol or a tertiary alcohol, the conversion rate was more likely to increase, the selection rate was also high (e.g., comparison between Example 1 and Example 2, or comparison between Example 5 and Example 6). It is thought that, when the alcohol had a branched chain, the side reaction was easily suppressed due to steric hindrance.

This application claims the priority to JP 2019-238725, filed on December 27, 2019. The contents described in the specification of said application are all incorporated herein by reference.

### [Industrial Applicability]

According to the method of producing an alkene of the present invention, an alkene, such as halogenated alkene, can be more efficiently produced. Therefore, the present invention is expected to contribute to the development and dissemination of the technologies, for example, in the field of synthesis involving an alkene such as halogenated alkene.

## Claims

1. A method of producing an alkene, comprising:
a step of contacting a solution containing an alcohol having three or more carbon atoms and a hydroxide of an alkali metal and/or an alkaline earth metal with a halogenated alkane.

2. The method of producing an alkene according to claim 1,
wherein the alcohol is water soluble.

3. The method of producing an alkene according to claim 1 or 2,
wherein a main chain of the alcohol is branched.

4. The method of producing an alkene according to claim 1 or 2,
wherein the alcohol is a secondary or tertiary alcohol.

5. The method of producing an alkene according to any one of claims 1 to 4,
wherein the alcohol contains two or more hydroxy groups.

6. The method of producing an alkene according to any one of claims 1 to 5, wherein the halogenated alkane is a compound represented by General Formula (1): 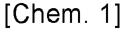 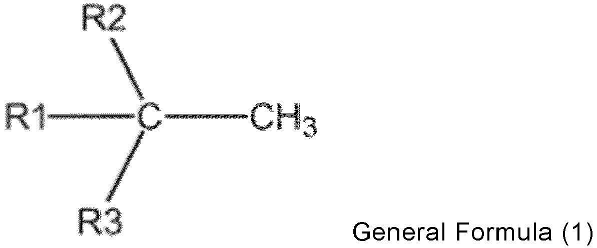 where, R1 represents a halogen atom, R2 represents a hydrogen atom, a halogen atom that is the same type as R1, or a halogen atom having a bond dissociation energy with a carbon atom greater than that of the atom represented by R1, R3 represents a halogen atom that is the same type as R1, a halogen atom having a bond dissociation energy with a carbon atom greater than that of the atom represented by R1, or an alkane group having from 1 to 3 carbon atoms which may be substituted with any halogen atom.

7. The method of producing an alkene according to claim 6,
wherein the halogenated alkane is a compound in which R1 in General Formula (1) represents a chlorine atom.

8. The method of producing an alkene according to any one of claims 1 to 7, wherein
the halogenated alkane is 1,1-difluoro-1-chloroethane, and
the contacting step is a step of producing 1,1-difluoroethylene.
